(19) Europäisches Patentamt
European Patent Office
Office européen des brevets

(11) Veröffentlichungsnummer: **0 034 114**
B1

# (12) EUROPÄISCHE PATENTSCHRIFT

(45) Veröffentlichungstag der Patentschrift: **09.02.83**

(51) Int. Cl.³: **C 07 J 11/00**, A 61 K 31/565 // C07J17/00, C07J33/00

(21) Anmeldenummer: **81730010.6**

(22) Anmeldetag: **02.02.81**

(54) **3-Desoxy-delta-15-Steroide, Verfahren zu ihrer Herstellung sowie diese enthaltende pharmazeutische Präparate.**

(30) Priorität: **11.02.80 DE 3005374**

(43) Veröffentlichungstag der Anmeldung: **19.08.81 Patentblatt 81/33**

(45) Bekanntmachung des Hinweises auf die Patenterteilung: **09.02.83 Patentblatt 83/6**

(84) Benannte Vertragsstaaten: **DE FR GB IT NL**

(56) Entgegenhaltungen:
**DE-A-1 934 272**
**FR-A-2 326 927**
**GB-A-1 070 943**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Hofmeister, Helmut, Dr., Weislingenstrasse 4, D-1000 Berlin 28 (DE)**
Erfinder: **Wiechert, Rudolf, Prof.Dr., Petzower Strasse 8A, D-1000 Berlin 39 (DE)**
Erfinder: **Annen, Klaus, Dr., Seegefelderstrasse 194, D-1000 Berlin 20 (DE)**
Erfinder: **Laurent, Henry, Dr., Glambecker Weg 21, D-1000 Berlin 28 (DE)**
Erfinder: **Beier, Sybille, Dr., Uhlandstrasse 121, D-1000 Berlin 31 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 034 114 B1

# 3-Desoxy-delta-15-Steroide, Verfahren zu ihrer Herstellung sowie diese enthaltende pharmazeutische Präparate

Die Erfindung betrifft 3-Desoxy-$\Delta^{15}$-Steroide, Verfahren zu ihrer Herstellung sowie diese enthaltende pharmazeutische Präparate.

Aus der FR-A-2 326 927 sind die entsprechenden 3-Oxosteroide bekannt, die wie die erfindungsgemäßen 3-Desoxysteroide der allgemeinen Formel I des Anspruchs 1 eine starke gestagene Wirkung besitzen. Die überlegene gestagene Wirkung der Erfindungsgemäßen neuen 3-Desoxysteroide gemäß Formel I war nicht vorauszusehen; denn die in GB-A-1 070 943 und DE-A-1 934 272 beschriebenen 3-Desoxysteroide, die sich von den Verbindungen der vorliegenden Anmeldung nur dadurch unterscheiden, daß sie keine Doppelbindung in 15-Stellung haben, zeigen eine viel geringere gestagene Wirksamkeit.

Die Acylgruppen $R^1$ gemäß Formel I leiten sich von Säuren ab, die in der Steroidchemie üblicherweise für Veresterungen angewendet werden. Bevorzugte Säuren sind organische Carbonsäuren mit bis zu 15 Kohlenstoffatomen, insbesondere niedere und mittlere aliphatische Carbonsäuren mit bis zu 7 Kohlenstoffatomen. Die Säuren können auch ungesättigt, verzweigt, mehrbasisch oder in üblicher Weise, zum Beispiel durch Hydroxy-, Acyloxy-, Alkoxy-, Oxo- oder Aminogruppen oder Halogenatome, substituiert sein. Geeignet sind auch cycloaliphatische, aromatische, gemischt aromatisch-aliphatische und hererocyclische Säuren, die ebenfalls in üblicher Weise substituiert sein können.

Beispielsweise seien folgende Carbonsäuren genannt:

Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, $\beta$-Cyclopentylpropionsäure, Cyclohexylessigsäure, Cyclohexancarbonsäure, Phenylessigsäure, Phenoxyessigsäure, Mono-, Di- und Trichloressigsäure, Aminoessigsäure, Diethylaminoessigsäure, Piperidinoessigsäure, Morpholinoessigsäure, Milchsäure, O-Tridecanoylglykolsäure, Bernsteinsäure, Adipinsäure, Benzoesäure, Nikotinsäure, Isonikotinsäure, Furan-2-carbonsäure.

Die neuen 3-Desoxy-$\Delta^{15}$-Steroide der allgemeinen Formel I werden gemäß Anspruch 11 durch Einführung des Restes $R^2$ in 18-Methyl-4,15-östradien-17-on der Formel II hergestellt.

Die Einführung des Restes $R^2$ kann nach bekannten Methoden mit einer metallorganischen Ethinyl-, Chlorethinyl- oder Propinylverbindung vorgenommen werden. Solche metallorganischen Verbindungen sind zum Beispiel Alkalimetallacetylide, wie zum Beispiel Kalium- und Lithiumacetylid, -chloracetylid bzw. -methylacetylid.

Die metallorganische Verbindung kann auch in situ gebildet und mit dem 17-Keton der Formel II zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines $C_4$- oder $C_5$-Alkohols oder von Ammoniak oder in Form von zum Beispiel Butyllithium einwirken lassen. Lithiumchloracetylid kann aus 1,2-Dichlorethylen und einer etherischen Lithiummethyllösung gebildet werden.

Als metallorganische Ethinylverbindungen sind auch Ethinylmagnesium- oder Ethinylzinkhalogenide, insbesondere Ethinylmagnesiumbromid oder -jodid, geeignet.

Als Lösungsmittel sind Dialkylether, Tetrahydrofuran, Dioxan, Benzol, Toluol usw., geeignet.

Die sich gegebenenfalls anschließende Veresterung der 17-Hydroxygruppe erfolgt nach Methoden, die man üblicherweise in der Steroidchemie zur Veresterung tertiärer Hydroxygruppen anwendet. Als geeignete Veresterungsmethode sei beispielsweise die Umsetzung der Steroide mit Säureanhydriden oder Säurechloriden in Gegenwart basischer Katalysatoren, wie Natriumhydrogenkarbonat, Kaliumhydrogenkarbonat, Kaliumkarbonat, Natriumhydroxid, Kaliumhydroxid, Pyridin, Lutidin, Collidin oder 4-Dimethylaminopyridin, genannt. Nach einer bevorzugten Methode wird die Veresterung in Gegenwart von Pyridin und 4-Dimethylaminopyridin durchgeführt. Das als Ausgangsmaterial des erfindungsgemäßen Verfahrens benutzte 18-Methyl-4,15-östradien-17-on der Formel II kann aus 3,3-Ethylendithio-15$\alpha$-hydroxy-18-methyl-4-östren-17-on (Deutsche Offenlegungsschrift 2 749 104) wie folgt hergestellt werden:

12,9 g 3,3-Ethylendithio-15$\alpha$-hydroxy-18-methyl-4-östren-17-on in 250 ml Tetrahydrofuran (THF) werden bei Raumtemperatur mit 25 ml Dihydropyran und 0,05 ml Phosphoroxichlorid versetzt. Nach 3 Stunden verdünnt man die Lösung mit Essigester, wäscht mit Wasser und trocknet. Nach Chromatographieren des Rohproduktes an Kieselgel mit 4,5–6% Aceton/Hexan werden 12,6 g 3,3-Ethylendithio-18-methyl-15$\alpha$-tetrahydropyran-2-yloxy-4-östren-17-on vom Schmelzpunkt 181,9°C erhalten.

1,0 g 3,3-Ethylendithio-18-methyl-15$\alpha$-tetrahydropyran-2-yloxy-4-östren-17-on in 20 ml THF werden

bei 0°C mit 2,5 ml Diisobutylaluminiumhydrid (20% Lösung in Toluol) versetzt. Nach 30 Minuten verdünnt man die Lösung mit Essigester, wäscht mit Wasser und trocknet. Es werden 900 mg 3,3-Ethylendithio-18-methyl-15α-tetrahydropyran-2-yloxy-4-östren-17β-ol als Öl erhalten.
$[\alpha]_D^{20} = +75°$.

Zu 250 ml flüssigem Ammoniak gibt man bei −70°C unter Rühren 1,4 g Lithium in kleinen Stücken. Nach 30 Minuten tropft man 6,5 g 3,3-Ethylendithio-18-methyl-15α-tetrahydropyran-2-yloxy-4-östren-17β-ol in 180 ml THF hinzu, rührt 1,5 Stunden und versetzt dann mit 100 ml Ethanol. Nach Abdampfen von Ammoniak löst man den Rückstand in Essigester, wäscht mit Wasser und trocknet im Vakuum.

Nach Chromatographieren des Rohproduktes (6,0 g) an Kieselgel mit 7,5−10% Aceton/Hexan werden 2,9 g 18-Methyl-15α-tetrahydropyran-2-yloxy-4-östren-17β-ol als Öl erhalten.
$[\alpha]_D^{20} = +69{,}4°$.

2,6 g 18-Methyl-15α-tetrahydropyran-2-yloxy-4-östren-17β-ol in 50 ml Methylenchlorid werden mit 3,6 g Pyridiniumchlorochromat und 1,2 g Natriumacetat bei 0°C gerührt. Nach 3,5 Stunden wird mit Methylenchlorid verdünnt, mit Wasser gewaschen und getrocknet. Nach Chromatographieren des Rohrproduktes an Kieselgel mit 3−3,5% Aceton/Hexan werden 1,6 g 18-Methyl-15α-tetrahydropyran-2-yloxy-4-östren-17-on als Öl erhalten.
$[\alpha]_D^{20} = +87{,}4°$.

28 g 18-Methyl-15α-tetrahydropyran-2-yloxy-4-östren-17-on in 250 ml Ethanol werden mit 30 ml halbkonzentrierter Salzsäure 2,5 Stunden bei Raumtemepratur gerührt. Das Gemisch wird mit Natriumhydrogencarbonatlösung neutralisiert und im Vakuum eingeengt. Den Rückstand löst man in Essigester, wäscht mit Wasser und Trocknet. Das Rohprodukt wird an Kieselgel chromatographiert. Mit 2−2,5% Aceton/Hexan werden 7,1 g 15α-Hydroxy-18-methyl-4-östren-17-on vom Schmelzpunkt 70,1°C eluiert.

7,8 g 15α-Hydroxy-18-methyl-4-östren-17-on in 50 ml Pyridin werden im Eisbad mit 10 ml Methansulfonsäurechlorid versetzt. Nach 1 Stunde gibt man 50 ml Dimethylformamid (DMF) und 23 g wasserfreies Natriumacetat hinzu und rührt weitere 20 Stunden bei Raumtemperatur. Das Gemisch wird in Eis/Wasser gegeben. Das ausgefallene Produkt wird abgesaugt, mit Wasser gewaschen, in Essigester gelöst und getrocknet. Nach Kristallisation aus Essigester werden 5,1 g 18-Methyl-4,15-östradien-17-on vom Schmelzpunkt 125,2°C erhalten.

Die neuen 3-Desoxy-$\Delta^{15}$-Steroide der allgemeinen Formel I sind pharmakologisch wirksame Substanzen. Sie zeigen ein ähnliches Wirkungsspektrum wie die in 3-Stellung oxygenierten Steroide und sind insbesondere gestagen wirksam. Beispielsweise zeigte das 17α-Ethinyl-18-methyl-4,15-östradien-17β-ol (A) im Clauberg-Test bei oraler Applikation an infantilen weiblichen Kaninchen eine der entsprechenden oxygenierten Verbindung vergleichbare gestagene Aktivität. Gleichzeitig wurde gefunden, daß die erfindungsgemäße Verbindung A im bekannten Levator ani-Samenblasen-Test an männlichen kastrierten Ratten nur geringe androgene Aktivität besitzt.

Im üblichen Clauberg-Test an infantilen weiblichen Kaninchen zeigt beispielsweise das erfindungsgemäße 17α-Ethinyl-18-methyl-4,15-östradien-17β-ol (A) gegenüber der anerkannt gut wirksamen Verbindung 17α-Ethinyl-18-methyl-4-östren-17β-ol-3-on (B) eine überlegene gestagene Wirksamkeit.

In der folgenden Tabelle werden die McPhail-Werte nach oraler Applikation angegeben.

Clauberg-Test

| Verbindung | Dosis (mg) | McPhail |
|---|---|---|
| A | 0,1 | 3,25 |
| | 0,03 | 2,5 |
| | 0,01 | 1,75 |
| B | 0,1 | 2,25 |
| | 0,03 | 1,5 |
| | 0,01 | 1,12 |

Beurteilung nach McPhail:

1 = keine Umwandlung des Endometriums;
4 = vollständige Umwandlung des Endometriums.

Die Verbindungen der allgemeinen Formel I können zum Beispiel in Antikonzeptionspräparaten Verwendung finden, wobei sie als Gestagenkomponente in Kombination mit einer östrogenwirksamen Hormonkomponente, wie zum Beispiel Ethinylöstradiol, oder als alleinige Wirkkomponente eingesetzt werden. Die Verbindungen können aber auch in Präparaten zur Behandlung gynäkologischer Störungen eingesetzt werden.

Zum Gebrauch werden die neuen Verbindungen mit den in der gelenischen Pharmazie üblichen Zusätzen, Trägersubstanzen und Geschmackskorrigentien nach an sich bekannten Methoden zu den üblichen Arzneimittelformen verarbeitet. Für die orale Applikation kommen insbesondere Tabletten, Dragées, Kapseln, Pillen, Suspensionen oder Lösungen in Frage. Für die parenterale Applikation kommen insbesondere ölige Lösungen, wie zum Beispiel Sesamöl- oder Rizinusöllösungen in Frage, die gegebenenfalls zusätzlich noch ein Verdünnungsmittel, wie zum Beispiel Benzylbenzoat oder Benzylalkohol, enthalten können. Die Konzentration des Wirkstoffes ist abhängig von der Applikationsform. So enthalten beispielsweise Tabletten zur oralen Applikation vorzugsweise 0,01 – 0,5 mg Wirkstoff und Lösungen zur parenteralen Applikation vorzugsweise 1 – 100 mg Wirkstoff pro 1 ml Lösung.

Die Dosierung der erfindungsgemäßen Arzneimittel kann sich mit der Form und dem Zweck der Verabfolgung ändern. Beispielsweise liegt die tägliche kontrazeptive Dosis bei oraler Applikation bei 0,01 – 0,5 mg.

## Beispiel 1

### 17α-Ethinyl-18-methyl-4,15-östradien-17β-ol

Acetylen wird 30 Minuten durch eine mit Eis/Wasser gekühlte Lösung von 15 ml Butyllithium (15%ig in Hexan) in 50 ml Tetrahydrofuran (THF) (absolut) geleitet. Unter Einleiten von Argon gibt man anschließend 500 mg 18-Methyl-4,15-östradien-17-on in 10 ml THF langsam hinzu. Nach 1 Stunde versetzt man mit gesättigter Ammoniumchloridlösung, verdünnt mit Ether, wäscht mit Wasser und trocknet. Es werden 450 mg 17α-Ethinyl-18-methyl-4,15-östradien-17β-ol als Öl erhalten.
Schmelzpunkt: 83,6° C (Pentan).

## Beispiel 2

### 17α-Chlorethinyl-18-methyl-4,15-östradien-17β-ol

Zu 1,1 ml 1,2-Dichlorethylen in 10 ml absolutem Ether werden unter Argon bei 0° C 7 ml einer 5%igen etherischen Methyllithiumlösung getropft. Nach 30 Minuten gibt man 430 mg 18-Methyl-4,15-östradien-17-on in 15 ml THF hinzu, rührt weitere 15 Minuten, versetzt mit gesättigter Ammoniumchloridlösung, verdünnt mit Ether und wäscht die Lösung mit Wasser. Das Rohprodukt wird an Kieselgel mit Aceton/Hexan chromatographiert. Es werden 310 mg 17α-Chlorethinyl-18-methyl-4,15-östradien-17β-ol als Öl erhalten.
$[\alpha]_D^{20} = -98°$.

## Beispiel 3

### 18-Methyl-17α-propin-1-yl-4,15-östradien-17β-ol

Methylacetylen wird etwa 30 Minuten durch eine mit Eis/Wasser gekühlte Lösung von 20 ml n-Butyllithium (15%ig in Hexan) in 80 ml absoluten THF geleitet. Anschließend tropft man unter Argon 800 mg 18-Methyl-4,15-östradien-17-on in 20 ml THF hinzu und rührt bei Raumtemperatur. Nach 20 Minuten versetzt man mit gesättigter Ammoniumchloridlösung, verdünnt mit Ether und wäscht mit Wasser. Das Rohprodukt wird an Kieselgel mit Aceton/Hexan chromatographiert. Es werden 540 mg 18-Methyl-17α-propin-1-yl-4,15-östradien-17β-ol als Öl erhalten.

## Beispiel 4

### 17β-Acetoxy-17α-ethinyl-18-methyl-4,15-östradien

1,0 g 17α-Ethinyl-18-methyl-4,15-östradien-17β-ol in 10 ml Pyridin werden bei Raumtemepratur unter Zugabe von 100 mg 4-Dimethylaminopyridin mit 7 ml Acetanhydrid umgesetzt. Das Gemisch wird nach 4 Stunden in Eis/Wasser gegeben. Das ausgefallene Produkt wird abgesaugt, in Essigester gelöst und mit Wasser gewaschen. Nach Chromatographieren des Rohproduktes an Kieselgel mit Aceton/Hexan erhält man 670 mg 17β-Acetoxy-17α-ethinyl-18-methyl-4,15-östradien als schaumiges Produkt.

Beispiel 5

17β-Butyryloxy-17α-ethinyl-18-methyl-4,15-östradien

230 mg 17α-Ethinyl-18-methyl-4,15-östradien-17β-ol in 3 ml Pyridin werden bei Raumtemperatur mit 1,5 ml Buttersäureanhydrid und 60 mg 4-Dimethylaminopyridin gerührt. Nach 5 Stunden gibt man das Gemisch in Eis/Wasser, extrahiert mit Essigester und wäscht mit Wasser. Das Rohprodukt wird an Kieselgel mit Essigester/Hexan chromatographiert. Es werden 160 mg 17β-Butyryloxy-17α-ethinyl-18-methyl-4,15-östradien als Öl erhalten.

Beispiel 6

17α-Ethinyl-17β-heptanoyloxy-18-methyl-4,15-östradien

300 mg 17α-Ethinyl-18-methyl-4,15-östradien-17β-ol in 4 ml Pyridin werden bei Raumtemperatur mit 2 ml Önanthsäureanhydrid und 60 mg 4-Dimethylaminopyridin 18 Stunden bei Raumtemperatur gerührt. Das Gemisch wird in Eis/Wasser gegeben. Es wird mit Essigester extrahiert und die Lösung mit Wasser gewaschen. Nach Chromatographieren des Rohproduktes an Kieselgel mit Essigester/Hexan erhält man 190 mg 17α-Ethinyl-17β-heptanoyloxy-18-methyl-4,15-östradien als Öl.

Beispiel 7

17β-Acetoxy-17α-chlorethinyl-18-methyl-4,15-östradien

570 mg 17α-Chlorethinyl-18-methyl-4,15-östradien-17β-ol in 6 ml Pyridin werden, wie in Beispiel 4 beschrieben, mit 3 ml Acetanhydrid und 50 mg 4-Dimethylaminopyridin umgesetzt. Nach Aufarbeitung und Chromatographieren mit Essigester/Hexan werden 390 mg 17β-Acetoxy-17α-chlorethinyl-18-methyl-4,15-östradien als schaumiges Produkt erhalten.

Beispiel 8

17β-Butyryloxy-18-methyl-17α-propin-1-yl-4,15-östradien

140 g 18-Methyl-17α-propin-1-yl-4,15-östradien-17β-ol in 2,5 ml Pyridin werden bei Raumtemperatur mit 1 ml Buttersäureanhydrid und 60 mg 4-Dimethylaminopyridin gerührt. Nach 8 Stunden wird die Lösung in Eis/Wasser gegeben. Es wird mit Essigester extrahiert, mit Wasser gewaschen und das Rohprodukt an Kieselgel mit Essigester/Hexan chromatographiert. Man erhält 65 mg 17β-Butyryloxy-18-methyl-17α-propin-1-yl-4,15-östradien als Öl.

Beispiel 9
(Zusammensetzung einer Tablette)

| | |
|---|---|
| 0,075 mg | 17α-Ethinyl-18-methyl-4,15-östradien-17β-ol |
| 0,030 mg | 17α-Ethinyl-östradiol |
| 109,895 mg | Milchzucker (DAB 6) |
| 8,000 mg | Maisstärke (USP XVI) |
| 1,000 mg | Magnesiumstearat (USP XVI) |
| 1,000 mg | Talkum |
| 120,000 mg | Gesamtgewicht der Tablette |

Beispiel 10
(Zusammensetzung eines Dragées)

| | |
|---|---|
| 0,100 mg | 17α-Ethinyl-18-methyl-4,15-östradien-17β-ol |
| 0,020 mg | 17α-Ethinyl-östradiol |
| 31,880 mg | Milchzucker |
| 18,425 mg | Maisstärke |
| 2,060 mg | Polyvinylpyrrolidon 25 |
| 0,010 mg | p-Oxybenzoesäuremethylester |
| 0,005 mg | p-Oxybenzoesäurepropylester |

2,500 mg Talkum
55,000 mg Gesamtgewicht der Tablette, die mit üblicher Zuckermischung auf etwa 90 mg dragiert wird.

**Patentansprüche**

1. 3-Desoxy-$\Delta^{15}$-Steroide der allgemeinen Formel I

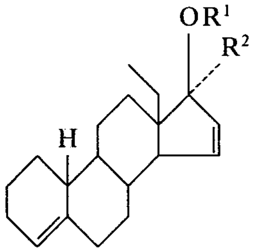

(I)

worin

$R^1$ ein Wasserstoffatom oder eine Acylgruppe und
$R^2$ eine Ethinyl-, Chlorethinyl- oder Propinylgruppe

bedeuten.

2. 17$\alpha$-Ethinyl-18-methyl-4,15-östradien-17$\beta$-ol.
3. 17$\alpha$-Chlorethinyl-18-methyl-4,15-östradien-17$\beta$-ol.
4. 18-Methyl-17$\alpha$-propin-1-yl-4,15-östradien-17$\beta$-ol.
5. 17$\beta$-Acetoxy-17$\alpha$-ethinyl-18-methyl-4,15-östradien.
6. 17$\beta$-Butyryloxy-17$\alpha$-ethinyl-18-methyl-4,15-östradien.
7. 17$\alpha$-Ethinyl-17$\beta$-heptanoyloxy-18-methyl-4,15-östradien.
8. 17$\beta$-Acetoxy-17$\alpha$-chlorethinyl-18-methyl-4,15-östradien.
9. 17$\beta$-Butyryloxy-18-methyl-17$\alpha$-propin-1-yl-4,15-östradien.
10. Pharmazeutische Präparate, gekennzeichnet durch einen Gehalt an Verbindungen gemäß Anspruch 1 – 9.
11. Verfahren zur Herstellung von 3-Desoxy-$\Delta^{15}$-Steroiden der allgemeinen Formel I

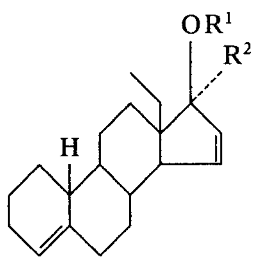

(I)

worin

$R^1$ ein Wasserstoffatom oder eine Acylgruppe und
$R^2$ eine Ethinyl-, Chlorethinyl- oder Propinylgruppe

bedeuten,

dadurch gekennzeichnet, daß man in das 17-Oxo-Steroid der Formel II

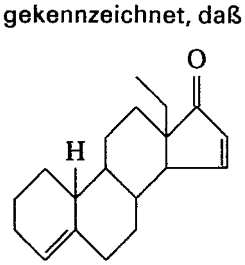

(II)

nach an sich bekannten Methoden mit Hilfe eines den Rest $R^2$ abgegebenen Mittels diesen Rest unter Bildung eines tertiären Carbinols am 17-C-Atom einführt und gegebenenfalls die 17-Hydroxygruppe verestert.

**Claims**

1. 3-Deoxy-$\Delta^{15}$-steroids of the general formula I

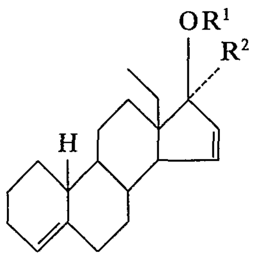

(I)

in which

$R^1$ represents a hydrogen atom or an acyl group, and
$R^2$ represents an ethynyl, chloroethynyl or propynyl group.

2. 17α-Ethynyl-18-methyl-4,15-oestradien-17β-ol.
3. 17α-Chloroethynyl-18-methyl-4,15-oestradien-17β-ol.
4. 18-methyl-17α-propyn-1-yl-4,15-oestradien-17β-ol.
5. 17β-Acetoxy-17α-ethynyl-18-methyl-4,15-oestradiene.
6. 17β-Butyryloxy-17α-ethynyl-18-methyl-4,15-oestradiene.
7. 17α-Ethynyl-17β-heptanoyloxy-18-methyl-4,15-oestradiene.
8. 17β-Acetoxy-17α-chloroethynyl-18-methyl-4,15-oestradiene.
9. 17β-Butyryloxy-18-methyl-17α-propyn-1-yl-4,15-oestradiene.
10. Pharmaceutical preparations characterised by a content of compounds according to claims 1 to 9.
11. Process for the manufacture of 3-deoxy-$\Delta^{15}$-steroids of the general formula I

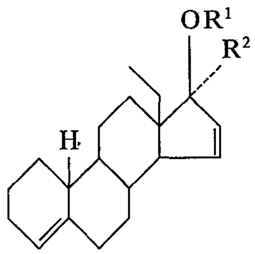

(I)

in which

$R^1$ represents a hydrogen atom or an acyl group, and
$R^2$ represents an ethynyl, chloroethynyl or propynyl group,

characterised in that the radical $R_2$ is introduced into the 17-oxo-steorid of the formula II

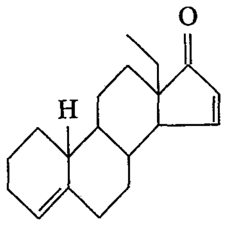

(II)

according to methods known per se, with the aid of an agent yielding the radical $R^2$, to form a tertiary carbinol at the 17-C atom, and, optionally, the 17-hydroxy group is esterified.

**Revendications**

1. Désoxy-3 $\Delta^{15}$ stéroïdes répondant à la formule générale I

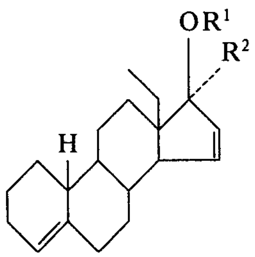

(I)

dans laquelle

$R^1$ représente un atome d'hydrogène ou un radical acycle et
$R^2$ représente un radical éthynyle, chloréthynyle ou propynyle.

2. Ethynyl-17α méthyl-18 oestradiène-4,15 ol-17β.
3. Chloréthynyl-17α méthyl-18 oestradiène-4,15 ol-17β.
4. Méthyl-18 (propyne-1 yl)-17α oestradiène-4,15 ol-17β.
5. Acétoxy-17β éthynyl-17α méthyl-18 oestradiène-4,15.
6. Butyryloxy-17β éthynyl-17α méthyl-18 oestradiène-4,15.
7. Ethynyl-17α heptanoyloxy-17β méthyl-18 oestradiène-4,15.
8. Acétoxy-17β chloréthynyl-17α méthyl-18 oestradiène-4,15.
9. Butyryloxy-17β méthyl-18 (propyne-1 yl)-17α oestradiène-4,15.
10. Compositions pharmaceutiques caractérisées en ce qu'elles contiennent des composés selon l'une quelconque des revendications 1 à 9.
11. Procédé de préparation de désoxy-3 $\Delta^{15}$-stéroïdes répondant à la formule générale I

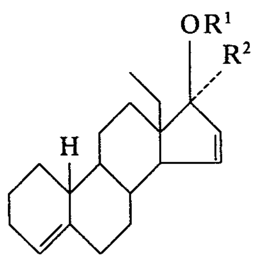

(I)

dans laquelle

$R^1$ représente un atome d'hydrogène ou un radical acycle et
$R^2$ un radical éthynyle, chloréthynyle ou propynyle,

procédé caractérisé en ce qu'on introduit un radical $R^2$ dans l'oxo-17 stéroïde de formule II

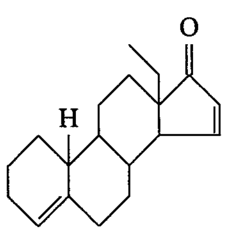

(II)

par des méthodes connues, au moyen d'un agent capable de céder un tel radical $R^2$, réaction qui conduit à un carbinol tertiaire à l'atome de carbone en 17, et éventuellement on estérifie le groupe hydroxy en 17.